# EUROPEAN PATENT APPLICATION

(11) **EP 1 541 186 A1**
(43) Date of publication of application: **15.06.2005**
(21) Application number: 04425915.8
(22) Date of filing: 13.12.2004
(51) Int. Cl.: A61M 11/06, B05B 7/00

(54) **Nebulizer device**

(30) Priority: 12.12.2003 IT BS20030075 U
(71) Applicant: FLAEM NUOVA S.p.A., I-25010 S. Martino Della Battaglia (Brescia) (IT)
(72) Inventor: Abate, Riccardo, 25100 S. Martino Della Battaglia (BR) (IT); Abate, Luigi, 25100 S. Martino Della Battaglia (BR) (IT)
(74) Representative: Sangiacomo, Fulvia

(57) **Abstract**

This invention concerns a atomizer chamber for atomizing of a medicament for aerosol therapy systems, which comprises a precipitator element (29) which is associated with an air inlet duct (19), which encompasses the atomizer nozzle (18) and forms a winding path for the nebulised liquid so as to compel the larger particles to drop and allow only the finer particles to move on towards the output duct (21). The precipitator element can be ring-shaped or have an elongation (35) which extends until it comes into contact with at least a part of the internal wall of the chamber below the output duct of the nebulised liquid.

## Description

### Field of Invention

This invention concerns the field of the equipment for atomizing of a medicament for the aerosol therapy, and refers more in particular to a nebuliser device chamber to be used with such equipment.

Differently made and shaped chambers which house an atomizer nozzle used to introduce a flow of nebulising air into a liquid medicine are already well-known in aerosol treatment.

The nebuliser device taken into consideration here, usually includes, in compliance with the preamble of claim 1:
a body which can be made up of two or more complementary parts which form a chamber made to receive a liquid medicine to be atomized,
a conduit, to introduce a flow of nebulising air, extending up into said chamber, with a top opening holding a nebuliser nozzle which has a crosspiece with a jet breaker cylinder, commonly known as an atomizer or pisper,
a through duct, open both towards the nebuliser nozzle and the exterior, for input of atmospheric air, and
a supply channel which extends from said chamber as far as an output duct of the nebuliser to the end user.

The output duct from which the atomized substance come out can be vertical, on an axis with the chamber, or at an angle and extending from one side of the chamber body, and a mouthpiece, a face mask or a nose adapter can be fitted to the output duct to facilitate inhaling of the nebulised substance on the part of the patient.

Atomization and mixing of the medicinal liquid with air is carried out by the atomizer nozzle. Here, a flow of air under pressure which arrives from below to the conduit entering from the bottom of the chamber, collaborates with the atmospheric air which enters the through conduit in the intake of the medicinal substance from said chamber and in its atomisation by means of the pisper.

However, the atomised substance at the level of the atomizer or pisper and then supplied through the output duct is not always uniform and fine as is wished for and required for efficient medicinal treatment of the respiratory tract. It is in fact well known that for some pathologies the finer the inhaled atomized medicinal drops are the more efficient is the type of treatment chosen.

### Objective of the invention

To meet this need and therefore to endeavour to resolve the problem of thinning and making the atomized substance uniform on exit from the atomizer chamber, a change has been made to the atomizer nozzle, changing its shape so that it is able to retain the larger medicinal drops or particles. Correspondingly, various atomizer chambers with an atomizer nozzle or pisper have been manufactured and modified in order to allow only the finer particles to reach the end user. One of these embodiments was, for example, described in a previous application for a patent by the same applicant, and carried out by associating the pisper with a deflector plate in the shape of a small umbrella.

An objective of this invention is however to resolve the problem mentioned above in a new and innovative way, as well as being simple and efficient, by inserting a precipitator element in the atomizer chamber that fits on the atmospheric air inlet duct and which in essence forms a ring shaped barrier around the pisper, forming a winding path for the atomized substance so as to compel the larger particles to drop into the chamber and allow only the finer particles to move on towards the output duct.

### Brief description of the invention

Further details of this invention will become more evident in the continuation of this description made in reference to the enclosed indicative and not limiting drawings, in which:
Fig. 1 shows a view from the side of an example of a chamber with vertical outlet;
Fig. 2 shows an top view of the chamber in Fig. 1;
Fig. 3 shows a cross section according to arrows A - A on Fig. 2 of a chamber housing a first embodiment of the precipitator.
Fig. 4 shows a similar section of a chamber with a vertical outlet housing a second embodiment of the precipitator;
Fig. 5 shows a side view of an example of a chamber with lateral outlet;
Fig. 6 shows a top view of the chamber in Fig. 5;
Fig. 7 shows a cross section according to arrows B - B on Fig. 6 of a chamber housing a first embodiment of the precipitator;
Fig. 8. shows a vertical section as in Fig. 7, but of a chamber housing a second embodiment of the precipitator;
Fig. 9 shows a side view of only the precipitator in its first form of construction:
Figs. 10 and 11 show top and bottom views of the precipitator in Fig. 9;
Fig. 12 shows a vertical section of the precipitator in Fig. 9
Figs. 13, 14 and 15 show respectively, a top view, a front view and a view only of the pisper;
Fig. 16 shows a top view of the pisper complete with precipitator;
Fig. 17 shows a cross section according to arrows E - E in Fig. 16; and
Fig. 18 shows a cross section according to arrows F - F on Fig. 17.

### Detailed description of the invention

In the examples shown, the chamber has a body 10 formed by two elements, one bottom 11 and one top 12, fitted together but separable, by means of coupling by screwing, by bayonet or some similar means 13,

The lower element 11 forms a chamber 14 for a medicinal liquid to be nebulised. This chamber has a bottom 15 with a duct 16 to allow air under pressure to enter, coming, according to arrow C, from a compressor. The duct 16 has a part 17 which enters the chamber 14 from the bottom to a point which is higher than the level of the medicinal liquid and which terminates in a conical portion with a top opening 17'. An atomizer nozzle 18 is assembled on the part extending upwards 17 of the air duct 16, and will be described later.

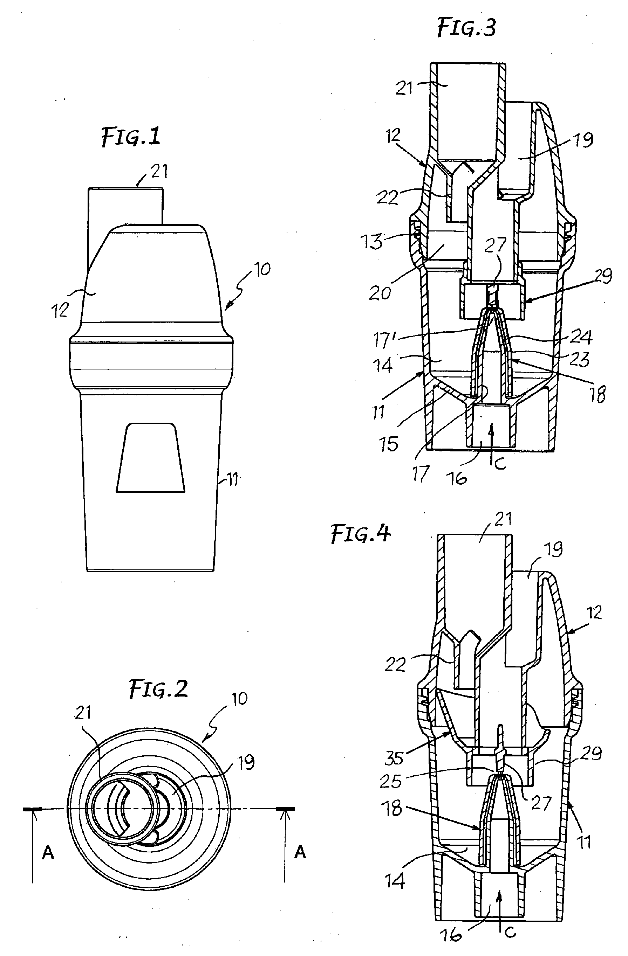

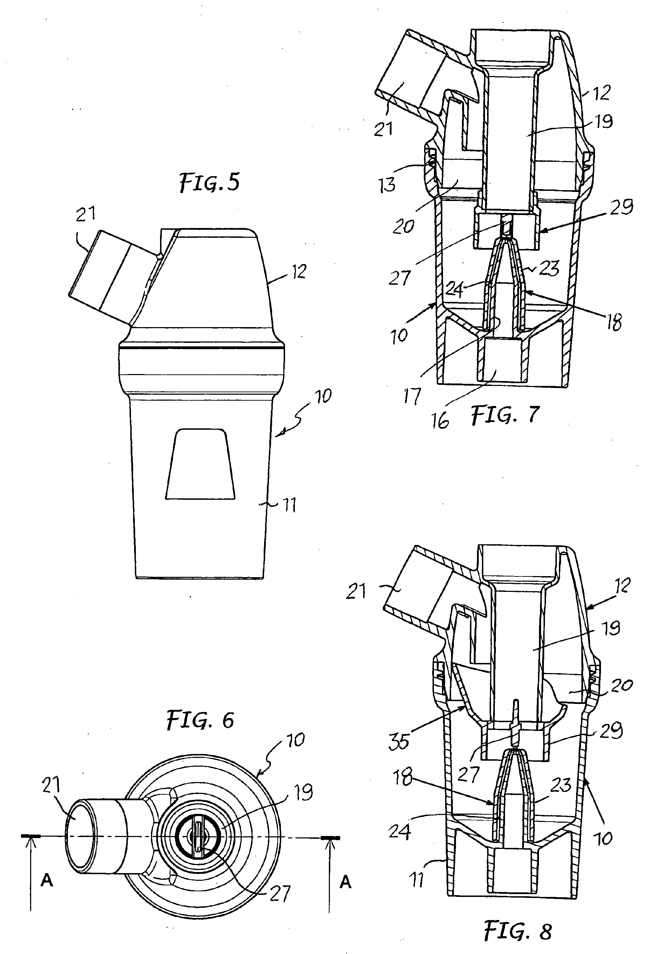

The top element 12 of the chamber body 10 has a through duct 19 to allow air to enter, open at the bottom in the direction of the nozzle 18 and at the top completely or only partially towards the outside, thanks to an exhaust valve - not shown. The top element 12, also forms a ring-shaped channel 12, around the through duct 19, open at the bottom towards the medicinal liquid chamber 14 and in communication on the other side with an outlet duct 21 which can be either vertical and exiting from the top pf the chamber body as in Figs. 1-4, or at an angle exiting from a side of the chamber body as shown in Figs. 5-8. In either case, the outlet duct 21 helps to cause the atomized substance to rise from the atomizer nozzle and which is fitted with a mouthpiece, a face mask or a nose adapter, according to needs, for inhalation of the atomized substance on the part of the patient. In addition, along the outlet duct 21 can be placed an anti-spill plate.

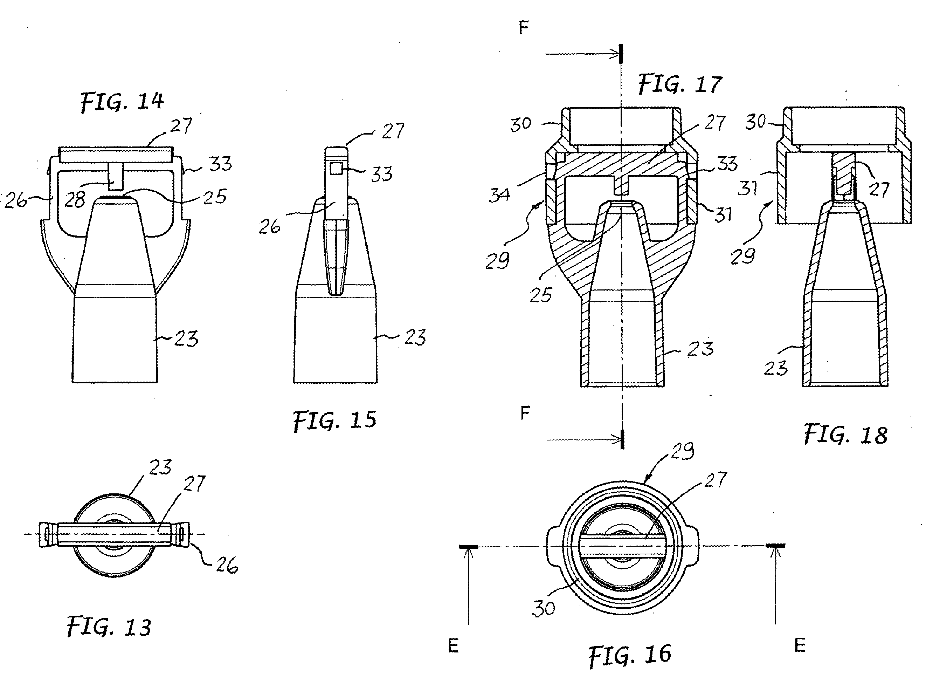

The atomizer nozzle 18 appears as -see Figs. 13-15 a cover 23 fitted on the part 17 of the duct 16 for the compressed air. The cover 23 and the part 17 of said duct 17 form together a ring-shaped cavity 24, which, is open radial at the bottom towards the chamber 14 of the medicinal liquid and communicating on the other part with an outlet duct 21 which, at the bottom is open radially and which draws from the medicinal liquid chamber 14 and, at the top, flows into injector nozzle 25 placed above and in line with the opening 17'. Two arms 26 extend upwards from the cover 23 which support a crosspiece 27 oriented crossways above the injector nozzle 25 and holding a jet breaker cylinder 28 facing towards the nozzle itself.

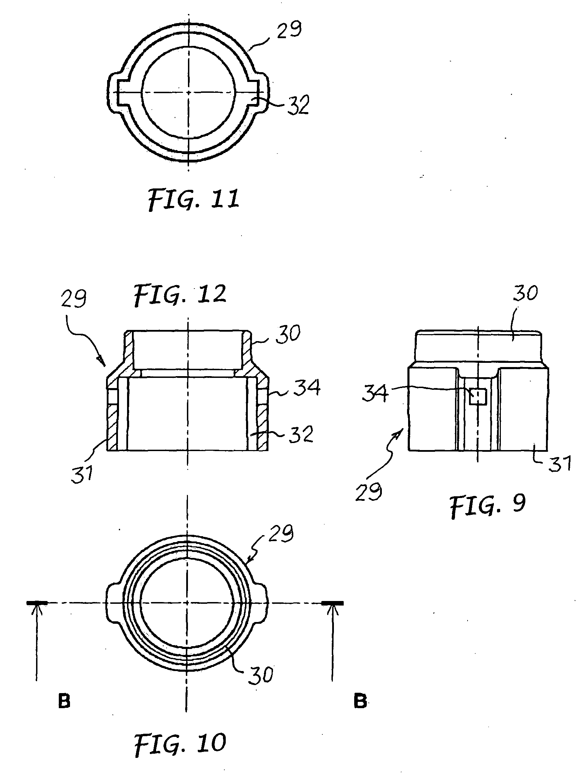

Now, in compliance with the invention, a precipitator element 29 is also provided associated with the atomizer nozzle 18 and the through duct 19. This precipitator element, in the example shown in Figs. 9-12, has a ring shape with one of its sections 30 made to couple with the bottom end of said through duct 19 and with another portion 31 which couples with the pisper 27. More precisely, this other portion 31 of the precipitator 29 has two internal grooves 32 which fit on the arms 26 that support the pisper 27, said arms having side locking tangs 33 which engages an opening 34 on the bottom of each groove 32 to attach the cover precipitator 23 as shown in Fig. 17 and so as to be able to remove and handle the two elements together.

In one construction variation, such as shown in Figs. 4 and 8, the precipitator element 29 is still in the ring-shaped configuration, but also has a lateral flared, elongation 35, in the shape of a glass, asymmetric, which extends towards the top in the annular channel 20 until it is in contact with at least one part of the internal surface of the top element 12 of the chamber body 10, so as to block the annular channel 20 at least in the part below the outlet duct 21 so as to avoid, in this way a direct passage of the nebulised liquid to the duct itself.

In the above described chamber, the air which arrives from below through duct 16 and which enters from the top through the through channel 19 causes the medicinal liquid to be drawn from the chamber 14 through the draw cavity. The medicinal liquid then flows towards the top zone of the Atomizer nozzle 18, where on a level with the openings 17', 18 the medicine and air mix, nebulising thanks to the obstacle in the form of the jet breaker or pisper 28.

The precipitator element 29, in either of the versions, substantially forms a barrier around the pisper 27 - see Figs. 4 and 8 - with the function of retaining the larger drops or particles of nebulised medicinal liquid, allowing the finer drops to move on towards the outlet duct 21.

The precipitator element 29 in itself, and even more so the one in the version with the flared elongation 35, forms, in fact, a winding path for the flow of the nebulised substance, which causes precipitation of the larger drops, and forms at the same time a drop collector, which avoids the drops coming into contact with the side walls of the chamber, encouraging them on the contrary to fall to the bottom of the latter.

## Claims

1. Atomizer chamber for aerosol therapy systems comprising:
- a body composed of two or more assembled, complementary parts and forming a chamber to contain a medicinal liquid to be nebulised,
- a duct entering and extending upwards in said chamber, used as an input for the nebuliser air flow, having an opening at the top and fitted with a nebuliser nozzle overhung by a crosspiece with jet breaker cylinder supported by two arms extending from said nozzle,
- a through duct, open both towards the nebuliser nozzle and towards the exterior for input of atmospheric air, and
- a supply channel which extends from said chamber up to a vertical or slanting outlet duct towards the user of the nebulised liquid which forms on a level with the crosspiece with jet breaker cylinder,
- **characterised by** a precipitator element (29) which fits into the through duct (19) of the atmospheric air inlet and which forms a ring-shaped barrier around the crosspiece with jet breaker cylinder (27, 28), forming a winding path for the nebulised liquid, compelling the larger particles to drop in the chamber, allowing only the finer drops to move on to the output duct.

2. Atomizer chamber according to claim 1, in which said precipitator element (29) is substantially ring-shaped with a part of it (30) used to fit to the bottom end of said through duct (19) and another section (31) which connects and encompasses said crosspiece with jet breaker cylinder (27, 28).

3. Atomizer chamber according to claim 1, in which, said precipitator element (29) is substantially ring-shaped with a part of it (30) designed to fit to the bottom end of said through duct (19) and another section (31) which connects and encompasses said crosspiece with jet breaker cylinder (27, 28), and a lateral flared, asymmetric elongation (35), which extends towards the top in the annular channel (20) until it is in contact with at least one part of the internal surface of the top element (12) of the chamber body (10), so as to block the annular channel (20) at least in the part below the outlet duct (21) of the nebulised liquid.

4. Atomizer chamber according to claims 2 or 3, in which said precipitator element (29) has, inside its section (31) encompassing the crosspiece with jet breaker cylinder (27, 28), two internal grooves (32) to couple with the supporting arms (26) of said crosspiece with jet breaker cylinder (27, 28), and where each of said arms has a lateral stop (33) to engage with an opening (34) at the bottom of each groove (32) to attach the precipitator element to the nebuliser nozzle.
